(19) Europäisches Patentamt
European Patent Office
Office européen des brevets

(11) EP 1 301 060 B1

## (12) EUROPÄISCHE PATENTSCHRIFT

(45) Veröffentlichungstag und Bekanntmachung des Hinweises auf die Patenterteilung:
**02.11.2005 Patentblatt 2005/44**

(51) Int Cl.⁷: **H04R 25/00**, A61B 5/12, H04R 29/00

(21) Anmeldenummer: **02017046.0**

(22) Anmeldetag: **29.07.2002**

(54) **Verfahren und Vorrichtung zur Erfassung von akustischen Parametern für die Anpassung von Hörhilfen**

Method and apparatus for determination of acoustic parameters for fitting hearing aids

Procédé et dispostif pour la détermination des paramètres acoustiques pour l'adaptation des prothèses auditives

(84) Benannte Vertragsstaaten:
**CH DE DK LI**

(43) Veröffentlichungstag der Anmeldung:
**09.04.2003 Patentblatt 2003/15**

(73) Patentinhaber: **PHONAK AG**
**8712 Stäfa (CH)**

(72) Erfinder: **Stirnemann, Alfred**
**8702 Zollikon (CH)**

(74) Vertreter: **Troesch Scheidegger Werner AG**
**Schwäntenmos 14**
**8126 Zumikon (CH)**

(56) Entgegenhaltungen:
**US-A- 4 289 143**       **US-A- 4 809 708**
**US-A- 5 526 819**

**Beschreibung**

Die vorliegende Erfindung betrifft ein Verfahren zur Erfassung von akustischen Parametern nach dem Oberbegriff von Anspruch 1 sowie einer Messvorrichtung nach dem Oberbegriff von Anspruch 8.

Das Ohr und der Gehörgang als Teil eines Organismus besitzen individuelle akustische Eigenschaften. Die Anpassung von Hörgeräten an diese individuellen akustischen Verhältnisse ist ein sehr komplizierter, aufwändiger und mit vielen Unsicherheiten behafteter Vorgang. Dabei spielen nach dem Einbringen eines Hörgerätes resp. einer Hörhilfe in den Gehörgang eine Reihe von Faktoren eine entscheidende Rolle, wie beispielsweise das Restvolumen im Gehörgang, die Distanz zum Trommelfell, die Trommelfellimpedanz, die Übertragungseigenschaften des Mittelohres, der Sitz der Hörhilfe, z.B. einer Otoplastik usw.

Herkömmlich werden akustische Impedanzmessungen im Ohr durchgeführt, aber praktisch ausschliesslich zu diagnostischen Zwecken, bekannt unter den Begriffen Tympanometrie, Reflektanzmessung, Compliancemessung etc. Derartige Messungen werden üblicherweise nur bei wenigen Frequenzen durchgeführt, wobei als Parameter der statische Druck im Ohrkanal variiert wird. Als Messeinrichtung wird dabei häufig die Anregung mittels eines Hörers und die Schallaufname mittels eines Mikrofons vorgenommen, welche entweder direkt oder über Adapter mit dem Gehörgang gekoppelt werden, wie beispielsweise aus der US 4,289,143 und der US 4,809,708 bekannt. Die Auswertung erfolgt schliesslich über externe, umfangreiche Elektronik, häufig mit Computerunterstützung, um die zu diagnostischen Zwecken interessierenden Resultate zu ermitteln. Aufgrund dieses Aufwandes und den damit verbundenen hohen Kosten eignet sich dieses Verfahren nicht für den Einsatz zur Anpassung von Hörhilfen.

Die Messung des effektiven Schalldruckes im Ohrkanal ist sehr aufwändig, und wird häufig mittels Schlauchsonden durchgeführt, welche sehr empfindlich auf die Endposition sind und bei der Messung auch das Schallsystem des Ohrkanals verändern. Insbesondere Messungen bei höheren Frequenzen sind daher mit grossen Unsicherheiten und Fehlerquellen behaftet, und aus den Resultaten lassen sich keine optimale Anpassungen für die Hörhilfen gewährleisten.

Die Aufgabe der vorliegenden Erfindung bestand nun darin, ein Verfahren zu finden, welches eine einfache und zuverlässige Anpassung der Hörhilfen ohne aufwändige und kosten- wie zeitintensive Messungen erlaubt.

Diese Aufgabe wird erfindungsgemäss durch die kennzeichnenden Merkmale nach Anspruch 1 gelöst. Weitere, bevorzugte Ausführungsformen ergeben sich aus den Merkmalen der weiteren Ansprüche 2 bis 7.

Weiter wird die Aufgabe durch eine Vorrichtung mit den kennzeichnenden Merkmalen nach Anspruch 8 gelöst. Weitere, bevorzugte Ausführungsformen dieser Vorrichtung ergeben sich aus den Merkmalen der weiteren Ansprüche 9 und 10.

Durch die Bestimmung der Impedanz über einen akustischen Referenzwiderstand hat weder der Frequenzgang des akustischen Empfängers noch die Signalfrequenz einen Einfluss auf das Messresultat. Damit wird vorteilhaft ein in Bezug auf die Frequenz breitbandiges Messsignal erhalten, welches direkt zur Auswertung herangezogen werden kann.

Ein grosser Vorteil besteht weiter darin, dass bei der bevorzugten Anordnung der Messorgane, d.h. der Mikrofone und des akustischen Widerstandes, im Gehäuse der Hörhilfe selbst keine zusätzlichen Messeinrichtungen notwendig sind, sondern die Messungen direkt mit der Hörhilfe selbst durchgeführt werden können. Damit entfallen insbesondere auch die Ungenauigkeiten der Messungen aufgrund der unterschiedlichen Verhältnisse im Gehörgang mit resp. ohne eingesetzte Hörhilfe.

Da herkömmlich bereits digitalisierte Technik in den miniaturisierten Hörhilfen, wie beispielsweise Im-Ohr-Hörgeräten, eingesetzt wird, muss selbst für die Auswerteeinrichtung kein grosser zusätzlicher Aufwand betrieben werden.

Ein weiterer Vorteil ist weiter darin zu sehen, dass die Messungen einfach periodisch wiederholt werden können, und damit die Hörhilfe periodisch den allenfalls veränderten Verhältnissen angepasst werden kann. Es ist durchaus denkbar, dass die Anpassung automatisch durch die Elektronik der Hörhilfe selbst durchgeführt werden kann, und damit keinen Eingriff von Aussen, ggf. durch einen Spezialisten, bedingt.

Durch das erfindungsgemässe Verfahren kann vorteilhaft eine optimale akustische Anpassung der Hörhilfen gewährleistet werden.

Ein Ausführungsbeispiel der vorliegenden Erfindung wird nachstehend anhand der beiliegenden Figur noch näher erläutert. Es zeigt

Fig. 1 schematisch den Längsschnitt durch eine erfindungsgemäss ausgestaltete Hörhilfe; und

Fig. 2 den gemessenen Verlauf der Impedanz einer Ohrschale in Amplitude und Phase.

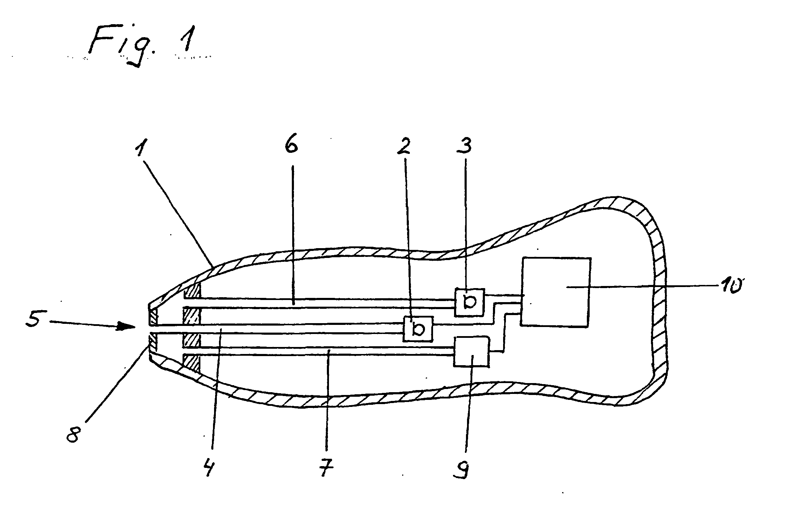

In Figur 1 ist schematisch der Längsschnitt durch eine Hörhilfe, hier beispielsweise eines miniaturisierten Hörgerätes, dargestellt. Der vordere Teil der in Form des äusseren Gehörganges gestalteten Geräteschale 1 wird in den Gehörgang (nicht dargestellt) eingeschoben. Innerhalb der Geräteschale 1 sind nun mindestens zwei Mikrofone 2 und 3 angeordnet. Das erste Mikrofon 2 ist über einen offenen Kanal 4 mit der Öffnung 5 der Geräteschale 1 verbunden. Dieses Mikrofon 2 dient der Messung des Schalldruckes im Gehörgang. Das zweite Mikrofon 3 ist ebenfalls über einen Kanal 6 mit der Öffnung 5 verbunden, wobei hier zwischen der Aussenseite der Öffnung 5 und dem Kanal 6 ein akustischer Widerstand 8 angebracht ist. Im vorliegenden Fall besteht der akustische Widerstand 8 vorzugsweise aus einer Kreisscheibe aus poröser Sinterbronze, und weist einen definierten, kalibrierten akustischen Widerstandswert $R_a$ auf.

Ein Hörer 9 liefert den verstärkten Aussenschall oder ein künstliches, breitbandiges Messsignal an den Kanal 7, welcher vor dem akustischen Widerstand 8 mit dem Kanal 6 zusammengeführt wird.

Die beiden Mikrofone 2 und 3 sind mit einer Auswertungselektronik 10 in Form eines Chips verbunden, welche aufgrund der nachfolgend beschriebenen Formel die Impedanz $Z_a$ berechnet.

$$Z_a = R_a * p_2/(p_1 - p_2)$$

Dabei stellen $p_2$ den zu messenden Schalldruck des Gehörganges (zwischen Hörhilfe und Trommelfell) und $p_1$ den Schalldruck vor dem akustischen Referenzwiderstand $R_a$ dar. Der Schalldruck wird mittels des jeweiligen Mikrofons 2 (Schalldruck $p_2$) resp. 3 (Schalldruck $p_1$) direkt gemessen, während der Widerstandswert $R_a$ über eine Kalibrierung des Widerstandes 8 aufgrund der bekannten Materialeigenschaften bestimmt ist.

Dadurch, dass der Schallfluss über die Druckdifferenz $p_1 - p_2$ bestimmt wird, kann die Bestimmung der Impedanz $Z_a$ vorteilhaft frequenzunabhängig erfolgen.

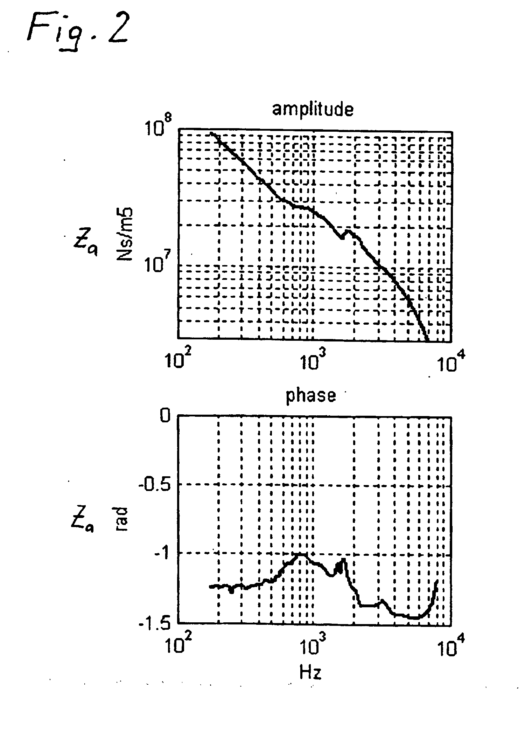

In Figur 2 ist als Beispiel das Resultat einer solchen erfindungsgemässen Messung resp. Berechnung der Impedanz $Z_a$ einer realen Ohrschale in Amplitude und Phase dargestellt.

Durch die Ausnützung der in der Hörhilfentechnik eingesetzten Technologie kann unter Verwendung von digitalen Rechnerchips ein breitbandiges Messresultat erlangt werden, wobei die Mikrofonsignale direkt ausgewertet und zur Berechnung der Impedanz herangezogen werden können.

Durch die Anwendung von curve-fit-Verfahren können die so gewonnenen Resultate zur Identifizierung einer Reihe von Mittelohrparametern herangezogen werden und beispielsweise folgende Eigenschaften berechnen oder zumindest abschätzen lassen :

- Komplexe Impedanz in einem Frequenzbereich bis zu ca. 10 kHz für optimale akustische Anpassung von Hörhilfen, insbesondere von Hörgeräten.

- Ermittlung der wirksamen Distanz zwischen Hörhilfenaustritt und Trommelfell.

- Compliance Restvolumen des Gehörganges.

- Gesamtcompliance bei tiefen Frequenzen, in Bezug auf das Restvolumen, das Trommelfell und das Mittelohr.

- Individuelle Mittelohrübertragungsfunktion.

- Wirkung von Vent und Undichtheiten in Bezug auf die Positionierung des Gehäuses der Hörhilfe.

Weiter können Anschlüsse für statische Druckänderungen vorgesehen werden, um einschlägige Tests aus der Tympanometrie durchzuführen. Ebenfalls können Aufzeichnungsfunktionen vorgesehen sein, welche bestimmte Parameter aufzeichnen, wie beispielsweise die Aufzeichnung von stimulierten otoakustischen Emissionen.

**Patentansprüche**

1. Verfahren zur Erfassung von akustischen Parametern des Ohres durch Messung der Impedanz des Gehörganges, **dadurch gekennzeichnet, dass** für die Bestimmung der Impedanz des Gehörganges ein erster Schalldruck mittels eines ersten Druckmikrofons (2) gemessen wird und der Schallfluss aus der Differenz des gemessenen ersten Schalldrucks in Bezug auf einen zweiten Schalldruck, welcher mittels eines zweiten Druckmikrofons (3) über einen kalibrierten akustischen Widerstand (8) gemessen wird, bestimmt wird, und aus dem ersten und zweiten Schalldruckwert schliesslich die Impedanz ($Z_a$) errechnet wird.

2. Verfahren nach Anspruch 1, **dadurch gekennzeichnet, dass** als akustischer Widerstand (8;$R_a$) eine poröse Sinterbronze eingesetzt wird, vorzugsweise konzentrisch um einen Kanal (4) des ersten Druckmikrofons (2) angeordnet.

3. Verfahren nach Anspruch 1 oder Anspruch 2, **dadurch gekennzeichnet, dass** die Messung mittels in einer Hörgeräteschale (1) angeordneter Druckmikrofone (2;3) erfolgt, wobei die Hörgeräteschale (1) vor der Messung in den Gehörgang eingeführt wird.

4. Verfahren nach Anspruch 3, **dadurch gekennzeichnet, dass** die Auswertung der Signale durch ebenfalls in der Hörgeräteschale angeordneter elektronischer Komponenten (10), vorzugsweise einem entsprechend programmierten oder programmierbaren Mikrochip, erfolgt.

5. Verfahren nach einem der Ansprüche 1 bis 4, **dadurch gekennzeichnet, dass** auf die Messresultate ein curve-fit-Verfahren angewendet wird, um folgende Parameter berechnen resp. zu schätzen :

- Impedanz, und/oder

- wirksame Distanz vom Hörgeräteaustritt zum Trommelfell, und/oder

- Compliance des Restvolumens des Gehörgangs, und/oder

- Gesamtcompliance bei tiefen Frequenzen, und/oder

- individuelle Mittelohrübertragungsfunktion, und/oder

- Wirkung von Undichtheiten.

6. Verfahren nach einem der Ansprüche 1 bis 5, **dadurch gekennzeichnet, dass** die Messung unter variablem, einstellbarem statischen Druck im Gehörgang durchgeführt wird.

7. Verfahren nach Anspruch 6, **dadurch gekennzeichnet, dass** der statische Druck über eine zusätzliche statische Druckleitung zum Gehörgang eingestellt wird.

8. Messvorrichtung zur Durchführung des Verfahrens nach einem der Ansprüche 1 bis 7, mit einer mindestens teilweise in den Gehörgang einzuführenden Sonde (1), **dadurch gekennzeichnet, dass** in der Sonde (1) zwei Druckmikrofone (2;3) zum Messen eines ersten und zweiten Schalldrucks angeordnet sind, wobei einem der beiden Druckmikrofone (3) ein akustischer Widerstand (8) vorgeschaltet ist.

9. Messvorrichtung nach Anspruch 8, **dadurch gekennzeichnet, dass** der akustische Widerstand (8) durch eine poröse Sinterbronze gebildet ist.

10. Messvorrichtung nach Anspruch 8 oder 9, **dadurch gekennzeichnet, dass** sie in der Geräteschale (1) einer Hörhilfe, vorzugsweise einer dem Gehörgang angepassten Geräteschale (1), angeordnet ist und vorzugsweise in der Hörhilfe eine Auswertungselektronik (10) angeordnet ist, welche mit den Mikrofonen (2,3) der Messvorrichtung verbunden ist.

**Claims**

1. Method for determination of acoustic parameters of the ear by measurement of the impedance of the auditory canal, **characterized in that** for determining the impedance of the auditory canal a first sound pressure is measured by means of a first sound pressure microphone (2) and by determining the sound flow by using the difference between said measured first sound pressure and a second sound pressure, measured by means of a second sound pressure microphone (3) through a calibrated acoustic resistance (8), and by finally calculating the impedance ($Z_a$) from said first and said second sound pressure values.

2. Method as claimed in claim 1, **characterized in that** a porous sinterbronge is used to form said acoustic resistance (8; $R_a$), which is preferably arranged concentrically around a canal (4) of said first sound pressure microphone (2).

3. Method as claimed in claim 1 or 2, **characterized in that** the measurement is carried out by means of sound pressure microphones (2, 3) arranged in a hearing aid casing (1), said hearing aid casing (1) being introduced into the authority canal before proceeding with the measurement.

4. Method as claimed in claim 3, **characterized in that** the computation of the signals is made by electronic components (10) also arranged in said hearing aid casing, preferably by a suitably programmed or programmable microchip.

5. Method as claimed in any of claims 1 to 4, **characterized in that** a curve-fit-method is applied on the measuring results for calculating or estimating the following parameters:

- impedance, and/ or
- effective distance from outlet of the hearing aid to the tympanic membrane, and/ or
- compliance of the residual volume of the auditory canal, and/ or
- total compliance at low frequencies, and/ or
- individual middle ear transmission function, and/ or
- effect of leaks.

6. Method as claimed in any of claims 1 to 5, **characterized in that** the measurement is carried out under a variable, adjustable static pressure in the auditory canal.

7. Method as claimed in claim 6, **characterized in that** the static pressure is adjusted through an additional static pressure pipe leading to the auditory canal.

8. Measuring apparatus for carrying out the method of any of claims 1 to 7, with a probe to be introduced at least partially into the auditory canal, **characterized in that** in said probe (1) two sound pressure microphones (2; 3) are arranged for measuring a first and a second sound pressure, whereby an acoustic resistance (8) is connected in series before one of said two sound pressure microphones (3).

**9.** Apparatus as claimed in claim 8, **characterized in that** said acoustic resistance (8) is formed by a porous sinterbronge.

**10.** Apparatus as claimed in claim 8 or 9, **characterized in that** it is arranged in the casing (1) of a hearing aid, preferably in a casing (1) adapted to the shape of auditory canal, and **in that** an electronic calculating unit (10) is preferably arranged in said hearing aid, connected to the microphones (2; 3) of the measuring apparatus.

**Revendications**

**1.** Procédé pour la détermination de paramètres acoustiques de l'oreille par mesurage de l'impédance du canal auditif, **caractérisé en ce que** l'on mesure pour la détermination de l'impédance du canal auditif une première pression acoustique au moyen d'un premier microphone à pression (2) et **en ce que** le flux de vitesse acoustiques est déterminé sur la base de la différence de ladite première pression acoustique mesurée par rapport à une seconde pression acoustique mesurée au moyen d'un second microphone à pression (3) à l'aide d'une résistance acoustique (8) calibrée et **en ce que** l'on calcule finalement l'impédance ($Z_a$) en utilisant les valeurs des premier et second pression acoustiques.

**2.** Procédé selon la revendication 1, **caractérisé en ce que** l'on utilise comme résistance acoustique (8; $R_a$) un bronze fritté qui est de préférence arrangé concentriquement autour d'un canal (4) dudit premier microphone à pression.

**3.** Procédé selon la revendications 1 ou 2, **caractérisé en ce que** le mesurage se fait au moyen de microphones à pression (2; 3) arrangé dans la coque (1) d'un appareil auditif, ladite coque (1) de l'appareil auditif étant introduit dans le canal auditif avant de procéder au mesurage.

**4.** Procédé selon la revendication 3, **caractérisé en ce que** l'exploitation des signaux se fait au moyen de composantes électroniques (10) également arrangées dans la coque de l'appareil auditif, de préférence au moyen d'un micro chip programmé ou programmable de manière appropriée.

**5.** Procédé selon l'une des revendications 1 à 4, **caractérisé en ce que** l'on applique sur les résultats du mesurage une méthode «curve-fit» pour calculer ou estimer les paramètres suivants:

- l'impédance et/ ou
- la distance effective de la sortie de l'appareil auditif au tympan, et/ ou
- la compliance du volume résiduel du canal auditif, et/ ou
- la compliance totale aux fréquences basses, et/ ou
- la fonction de transmission individuelle de l'oreille moyenne, et/ ou
- l'effet de fuites.

**6.** Procédé selon l'une des revendications 1 à 5, **caractérisé en ce que** le mesurage se fait sous une pression statique variable et réglable dans le canal auditif.

**7.** Procédé selon la revendication 6, **caractérisé en ce que** la pression statique est réglée à travers une conduite de pression statique supplémentaire menant au canal auditif.

**8.** Dispositif de mesure pour la mise en oeuvre du procédé selon l'une des revendications 1 à 7, comprenant une sonde (1) qui est au moins partiellement à introduire dans le canal auditif, **caractérisé en ce qu'**au moins deux microphones à pression (2; 3) sont arrangés dans la sonde (1) pour mesurer une première et une seconde pression acoustique, une résistance acoustique (8) étant arrangée en série devant l'un des deux microphones à pression (3).

**9.** Dispositif de mesure selon la revendication 8, **caractérisé en ce que** la résistance acoustique (8) est formé par un bronze fritté poreux.

**10.** Dispositif de mesure selon la revendication 8 ou 9, **caractérisé en ce qu'**il est arrangé dans la coque (1) d'un appareil auditif, de préférence dans la coque (1) d'un appareil auditif adapté à la forme du canal auditif, et **en ce que** de préférence une unité d'évaluation électronique (10) est arrangée dans l'appareil auditif connecté aux microphones (2; 3) du dispositif de mesure.

Fig. 1

Fig. 2